(19) Europäisches Patentamt
European Patent Office
Office européen des brevets

(11) **EP 4 666 867 A1**

(12) **EUROPEAN PATENT APPLICATION**
published in accordance with Art. 153(4) EPC

(43) Date of publication:
**24.12.2025 Bulletin 2025/52**

(21) Application number: **24756906.4**

(22) Date of filing: **14.02.2024**

(51) International Patent Classification (IPC):
*A23L 5/00* (2016.01)    *A23C 11/00* (2025.01)
*C12N 9/28* (2006.01)    *C12N 9/42* (2006.01)
*A23L 7/104* (2016.01)

(52) Cooperative Patent Classification (CPC):
A23C 11/00; A23L 5/00; C12N 9/2414;
C12N 9/2434; A23L 7/104

(86) International application number:
**PCT/JP2024/004985**

(87) International publication number:
**WO 2024/172065 (22.08.2024 Gazette 2024/34)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **15.02.2023 JP 2023021830**

(71) Applicants:
• **Amano Enzyme Inc.**
**Nagoya-shi**
**Aichi 460-8630 (JP)**

• **Amano Enzyme USA Co., Ltd.**
**Elgin, IL 60124 (US)**

(72) Inventor: **HASHIMURA, Dai**
**Elgin, Illinois 60124 (US)**

(74) Representative: **Mullholland, Lewis Paul**
**WP Thompson**
**44 Southampton Buildings**
**London WC2A 1AP (GB)**

(54) **ENZYME AGENT FOR PRODUCING VISCOUS PLANT-BASED FOOD OR BEVERAGE**

(57)    [Summary]

[Problems] To provide a technique for enhancing the viscosity of plant-based viscous foods and drinks.

[Solution] The present technique provides a plant-based viscous food and drink-producing enzyme preparation, which contains $\alpha$-amylase and cellulase. The plant-based viscous food and drink-producing enzyme preparation of the present technique can be used as a plant-based fermented food and drink-producing enzyme preparation or a plant-based lactic acid fermented food and drink-producing enzyme preparation. The present technique also provides a viscosity enhancer for plant-based viscous food and drink, which contains $\alpha$-amylase and cellulase. The present technique further provides a method for producing plant-based viscous foods and drinks, and a method for enhancing the viscosity of plant-based viscous foods and drinks, which include a step of allowing $\alpha$-amylase and cellulase to act on a plant-based raw material.

EP 4 666 867 A1

## Description

Technical Field

The present technique relates to a plant-based viscous food and drink-producing enzyme preparation. More specifically, the present technique relates to a plant-based viscous food and drink-producing enzyme preparation that can enhance the viscosity of a plant-based viscous food and drink, a viscosity enhancer for plant-based viscous food and drink, a method for producing a plant-based viscous food and drink, and a method for enhancing the viscosity of a plant-based viscous food and drink.

Background Art

In recent years, the food and drink market has seen the market for plant-based foods and drinks as dairy and meat substitute markets expanding driven by a focus on sustainability and health consciousness. As an example of plant-based foods and drinks, for example, plant-based yogurt occupies an important category in the dairy substitute market and continues to be a field that attracts attention. In the production of a plant-based yogurt, the standard method is to mix carbohydrate sources, protein sources, and lipid sources with plant-based milk as needed and then ferment the mixture with lactic acid bacteria.

Under such a background, various techniques have been developed in recent years for the purpose of improving the physical properties of plant-based foods and drinks. For example, Patent Literature 1 discloses a method for producing a plant-based yogurt by fermenting cereal starch hydrolyzed by an enzyme produced by koji mold or an enzyme preparation derived from koji mold, with lactic acid bacteria. Patent Literature 2 discloses a method for producing an oat base, a non-dairy yogurt and the like with enhanced viscosity by allowing transglutaminase to act on an oat base treated with $\alpha$- or $\beta$-amylase. Patent Literature 3 discloses a method for producing a plant-based protein food (particularly a plant-based yogurt) with improved flavor and properties by allowing a protein deamidating enzyme and a lipase to act on a plant-based protein raw material. Non-Patent Literature 1 discloses that the texture, sensory, nutritional and functional properties of a plant-based yogurt are improved by using an exopolysaccharide (EPS)-producing bacterium as a starter for fermenting a plant-based yogurt.

Citation List

Patent Literature

Patent Literature 1: International Publication WO2022/037920
Patent Literature 2: International Publication WO2017/171601
Patent Literature 3: International Publication WO2022/014542

Non-Patent literature

Non-Patent Literature 1: Manufacture and characterization of a yogurt-like beverage made with oat flakes fermented by selected lactic acid bacteria, International Journal of Microbiology 185 (2014) 17-26.

Summary of Invention

Technical Problem

Plant-based foods and drinks differ from animal-based foods and drinks in that (1) they contain less protein, (2) they have different coagulation properties, and (3) they require the addition of structuring agents and emulsifiers, which often result in time-consuming and costly processes in the production methods thereof, and thus, there is still room for improvement before satisfactory characteristics can be obtained. In particular, when viscous foods and drinks that require viscosity are produced using plant-based raw materials, additives such as thickeners and emulsifiers used to enhance viscosity do not fit the concept of clean label products, and therefore, replacements are desired.

Therefore, the main objective of this technique is to provide a novel technique for enhancing the viscosity of a plant-based viscous food and drink.

Solution to Problem

As a result of extensive research, the inventors of the present application have succeeded in enhancing the viscosity of viscous food and drink products produced from plant-based raw materials by allowing $\alpha$-amylase and cellulase to act on the plant-based raw materials. Based on this knowledge, the present technique has been completed through further research.

That is, the present technique first provides a plant-based viscous food and drink-producing enzyme preparation, which contains $\alpha$-amylase and cellulase.

The plant-based viscous food and drink-producing enzyme preparation according to the present technique can be used in the production of plant-based fermented foods and drinks.

Furthermore, the plant-based viscous food and drink-producing enzyme preparation according to the present technique can be used in the production of plant-based lactic acid fermented foods and drinks.

Furthermore, the plant-based viscous food and drink-producing enzyme preparation according to the present technique can be used in the production of a plant-based yogurt.

Next, the present technique provides a viscosity enhancer for plant-based viscous food and drink, which contains $\alpha$-amylase and cellulase.

The present technique further provides a method for producing a plant-based viscous food and drink, which includes a step of allowing $\alpha$-amylase and cellulase to act on a plant-based raw material, and a method for enhancing the viscosity of a plant-based viscous food and drink, which includes a step of allowing $\alpha$-amylase and cellulase to act on a plant-based raw material.

Advantageous Effects of Invention

According to the present technique, the viscosity of a plant-based viscous food and drink can be enhanced without using additives.

Description of Embodiments

A preferred embodiment for carrying out the present technique will be described below. Note that the embodiment described below shows an example of a typical embodiment of the present technique, and the scope of the present technique is not to be interpreted narrowly by this.

1. Plant-based viscous food and drink-producing enzyme preparation, viscosity enhancer for plant-based viscous food and drink

The plant-based viscous food and drink-producing enzyme preparation and the viscosity enhancer for plant-based viscous food and drink according to the present technique contain $\alpha$-amylase and cellulase. As will be demonstrated in the examples described below, the present technique has succeeded in dramatically enhancing the viscosity of a produced plant-based viscous food and drink by treating a plant-based raw material with $\alpha$-amylase and cellulase during the production of the plant-based viscous food and drink. In addition, as will be demonstrated in the examples described below, the water retention of a plant-based viscous food and drink produced was also successfully enhanced by treating a plant-based raw material with $\alpha$-amylase and cellulase during the production of the plant-based viscous food and drink. Below, components and the like that can be used in the plant-based viscous food and drink-producing enzyme preparation and the viscosity enhancer for plant-based viscous food and drink according to the present technique will be described in detail.

(1) $\alpha$-Amylase

$\alpha$-Amylase is an enzyme that acts on starch and mainly hydrolyzes $\alpha$-1,4-glycosidic bond. The $\alpha$-amylase that can be used in the present technique may be an enzyme that further has other actions as long as it has $\alpha$-amylase activity.

The origin of $\alpha$-amylase that can be used in the present technique is not particularly limited as long as it does not impair the action and effect of the present technique. Examples thereof include filamentous fungi including Aspergillus aureus, Aspergillus foetidus, Aspergillus niger, and Aspergillus oryzae; actinomycetes including Saccharomonospora viridis, Streptomyces avermitilis, Streptomyces griseus, Streptomyces thermoviolaceus, Streptomyces violaceoruber, and Thermomonospora viridis; and bacteria including Alcaligenes latus, Arthrobacter, Bacillus amyloliquefaciens, Bacillus circulans, Bacillus licheniformis, Bacillus stearothermophilus, Bacillus subtilis, Cellulosimicrobium cellulans, Microbacterium imperiale, Paenibacillus alginolyticus, and Sulfolobus solfataricus. Preferred is an $\alpha$-amylase derived from the genus Bacillus or an $\alpha$-amylase derived from Aspergillus, and more preferred is an $\alpha$-amylase derived from

Bacillus amyloliquefaciens or an α-amylase derived from Bacillus licheniformis.

In the present technique, not only natural (wild-type) α-amylase but also recombinant α-amylase can be used. Commercially available α-amylase or α-amylase preparations can also be used. An example of a commercially available α-amylase or α-amylase preparation is α-amylase derived from Bacillus amyloliquefaciens manufactured by Amano Enzyme Inc.

The α-amylase used in the present technique can be prepared from a culture solution of a microorganism from which the above-mentioned cellulase is derived. Specific preparation methods include a method of recovering α-amylase from the culture solution or bacterial bodies of the above-mentioned microorganism. For example, when an α-amylase-secreting microorganism is used, the bacterial bodies can be recovered from the culture solution in advance by filtration, centrifugation, or the like as necessary, and the enzyme can then be separated and/or purified. When an α-amylase non-secreting microorganism is used, the bacterial bodies can be recovered from the culture solution in advance as necessary, then the bacterial bodies can be crushed by pressure treatment, ultrasonic treatment, or the like to extract the enzyme, and the enzyme can then be separated and/or purified. As a method for separating and/or purifying the enzyme, a known protein separation and/or purification method can be used without any particular limitation, and examples of the method include centrifugation, UF concentration, salting out, and various chromatography methods using ion exchange resins, etc. The separated and/or purified enzyme can be powdered by a drying method such as freeze-drying or vacuum drying, and can also be powdered using an appropriate excipient and/or drying aid in the drying method. In addition, the separated and/or purified enzyme can be liquefied by adding appropriate additives and sterilizing by filtration.

The content of α-amylase in the plant-based viscous food and drink-producing enzyme preparation according to the present technique and the viscosity enhancer for plant-based viscous food and drink according to the present technique is not particularly limited as long as it does not impair the action and effect of the present technique. The lower limit of the content of α-amylase can be set to, for example, 0.5 U or more, preferably 1 U or more, 5 U or more, 10 U or more, more preferably 20 U or more, 30 U or more, 40 U or more, 50 U or more, and even more preferably 60 U or more, 70 U or more, 80 U or more, or 90 U or more per 1 g of the plant-based raw material of the plant-based viscous food and drink. In addition, the lower limit of the content of α-amylase can be set to, for example, 1 U or more, preferably 2 U or more, 5 U or more, 10 U or more, more preferably 20 U or more, 40 U or more, and even more preferably 60 U or more, 80 U or more, 100 U or more, or 120 U or more per 1 g of carbohydrate (preferably starch) contained in the plant-based raw material of the plant-based viscous food and drink.

On the other hand, the upper limit of the content of α-amylase can be set to, for example, 500,000 U or less, 100,000 U or less, 50,000 U or less, 10,000 U or less, 5,000 U or less, preferably 4,000 U or less, 2,000 U or less, 1,000 U or less, more preferably 800 U or less, 600 U or less, 400 U or less, 200 U or less, 120 U or less, per 1 g of plant-based raw material of the plant-based viscous food and drink. In addition, the upper limit of the content of α-amylase can be set to, for example, 700,000 U or less, 150,000 U or less, 70,000 U or less, 15,000 U or less, 7,000 U or less, preferably 6,000 U or less, 3,000 U or less, 1,500 U or less, more preferably 1,000 U or less, 800 U or less, 600 U or less, 300 U or less, 200 U or less, or 150 U or less per 1 g of carbohydrates (preferably starch) contained in the plant-based raw material of the plant-based viscous food and drink.

In this technique, regarding the activity of α-amylase, the amount of enzyme that reduces the color of potato starch caused by iodine by 10% in one minute when treated with 1% potato starch as a substrate at pH 5.0 and 37°C is defined as 1 unit (1 U). In this technique, the activity of α-amylase is a value measured by the α-amylase activity measurement method described in the Examples below.

(2) Cellulase

Cellulase is an enzyme that hydrolyzes the glucosidic bond of β-1,4 glucan. The cellulase that can be used in the present technique may be an enzyme that further has other actions as long as it has cellulase activity.

The origin of cellulase that can be used in the present technique is not particularly limited as long as it does not impair the action and effect of the present technique. Examples thereof include cellulases derived from: basidiomycetes including the genus Corticium, the genus Irpex, and Pycnoporus coccineus; filamentous fungi including Acremonium cellulolyticus, Aspergillus aculeatus, Aspergillus awamori, Aspergillus niger, Humicola insolens, Penicillium funiculosum, Trichoderma harzianum, Trichoderma insolens, Trichoderma koningii, Trichoderma longibrachiatum, Trichoderma reesei, and Trichoderma viride; actinomycetes including those of the genus Actinomyces and Streptomyces; and bacteria including Bacillus circulans and Bacillus subtilis. Preferably, cellulases are derived from microorganisms of the genus Trichoderma. Examples of the microorganisms belonging to the genus Trichoderma include Trichoderma reesei and Trichoderma viride. Preferably, the cellulase is derived from Trichoderma viride.

In this technique, not only natural (wild-type) cellulase but also recombinant cellulase can be used. Commercially available cellulases or cellulase preparations can also be used. Examples of commercially available cellulases or cellulase preparations include cellulases derived from Trichoderma viride manufactured by Amano Enzyme Inc. as cellulases derived from microorganisms of the genus Trichoderma, and cellulases derived from Aspergillus niger manufactured by Amano Enzyme Inc. as cellulases derived from microorganisms of the genus Aspergillus.

The cellulase used in the present technique can be prepared from the culture solution of the microorganism from which the above-mentioned cellulase is derived. Specific preparation methods include a method of recovering cellulase from the culture solution or bacterial bodies of the above-mentioned microorganism. For example, when a cellulase-secreting microorganism is used, the bacterial bodies can be recovered from the culture solution in advance by filtration, centrifugation, etc., as necessary, and then the enzyme can be separated and/or purified. In addition, when a cellulase non-secreting microorganism is used, the bacterial bodies can be recovered from the culture solution in advance as necessary, then the bacterial bodies can be crushed by pressure treatment, ultrasonic treatment, etc. to extract the enzyme, and then the enzyme can be separated and/or purified. As a method for separating and/or purifying the enzyme, a known protein separation and/or purification method can be used without any particular limitation, and examples of the method include centrifugation, UF concentration, salting out, various chromatography methods using ion exchange resins, etc. The separated and/or purified enzyme can be powdered by a drying method such as freeze-drying or vacuum drying, and can also be powdered using an appropriate excipient and/or drying aid in the drying method. In addition, the separated and/or purified enzyme can be liquefied by adding appropriate additives and sterilizing by filtration.

The content of cellulase in the plant-based viscous food and drink-producing enzyme preparation according to the present technique and the viscosity enhancer for plant-based viscous food and drink according to the present technique is not particularly limited as long as it does not impair the action and effect of the present technique. The lower limit of the content of cellulase can be set to, for example, 0.01 U or more, preferably 0.1 U or more, 0.5 U or more, 1 U or more, more preferably 2 U or more, 5 U or more, even more preferably 10 U or more, 15 U or more, or 20 U or more per 1g of the plant-based raw material of the plant-based viscous food and drink. In addition, the lower limit of the content of cellulase can be set to, for example, 0.1 U or more, preferably 1 U or more, 5 U or more, 10 U or more, more preferably 20 U or more, 50 U or more, even more preferably 100 U or more, 150 U or more, or 180 U or more per 1g of dietary fiber contained in the plant-based raw material of the plant-based viscous food and drink.

On the other hand, the upper limit of the content of cellulase can be set to, for example, 10,000 U or less, 5,000 U or less, 2,000 U or less, 1,000 U or less, 500 U or less, preferably 200 U or less, 100 U or less, more preferably 80 U or less, 60 U or less, 40 U or less, 30 U or less, or 25 U or less per 1 g of the plant-based raw material of the plant-based viscous food and drink. Also, the upper limit of the content of cellulase can be set to, for example, 100,000 U or less, 50,000 U or less, 20,000 U or less, 10,000 U or less, 5000 U or less, preferably 2000 U or less, 1000 U or less, more preferably 800 U or less, 600 U or less, 400 U or less, 300 U or less, or 250 U or less per 1 g of dietary fiber contained in the plant-based raw material of the plant-based viscous food and drink.

In this technique, regarding the activity of cellulase, the amount of enzyme that produces reducing sugars equivalent to 1 $\mu$mole of glucose per minute when treated with sodium carmellose (also known as sodium carboxymethylcellulose) as a substrate at pH 4.5 and 37°C is defined as 1 unit (1U). In this technique, the activity of cellulase is a value measured by the cellulase activity measurement method described in the Examples below.

The blending ratio of $\alpha$-amylase and cellulase in the plant-based viscous food and drink-producing enzyme preparation and the viscosity enhancer for plant-based viscous food and drink according to the present technique is not particularly limited as long as it does not impair the action and effect of the present technique. The blending ratio of $\alpha$-amylase and cellulase in the plant-based viscous food and drink-producing enzyme preparation and the viscosity enhancer for plant-based viscous food and drink according to the present technique can be set, for example, as follows: $\alpha$-amylase activity:cellulase activity = 1:10 to 100:1, preferably 1:1 to 50:1, 2:1 to 20:1, 3:1 to 10:1, 4:1 to 6:1, 5:1 to 3:1, more preferably 1:2 to 100:1, 1:1.5 to 50:1, 1:1.2 to 30:1, 1:1.2 to 20:1, or 1:1.2 to 10:1.


(3) Plant-based raw material

As plant-based raw materials in which the plant-based viscous food and drink-producing enzyme preparation and the viscosity enhancer for plant-based viscous food and drink according to the present technique can be used, one or more types of plant-based raw materials that can be used for viscous foods and drinks can be freely selected and used, as long as the action and effect of the present technique is not impaired. Examples of the raw materials include cereals such as wheat, barley, oats, rice, rye, buckwheat, barnyard millet, millet, and teff; pulses such as soy beans, green peas, lentils, chickpeas, black beans, broad beans, mung beans, lupine beans, and kidney beans; nuts such as almonds, coconuts, peanuts, cashew nuts, hazelnuts, pecan nuts, macadamia nuts, pistachios, walnuts, Brazil nuts, pili nuts, chestnuts, sesame seeds, and pine nuts; and hemp seeds (industrial hemp), chia seeds, quinoa, amaranth, canary seeds, and linseeds. In the present technique, among these raw materials, preferred are cereals and pulses other than soybeans, and more preferred are oats.

The starch content (based on the weight of the plant-based raw material in a dried state) contained in the plant-based raw material is not particularly limited, and a plant-based raw material containing a preferred amount of starch can be selected depending on the type of viscous food and drink to be produced and the desired physical properties. In the present technique, for example, a plant-based raw material containing starch of 1% by weight or more, preferably 10% by weight or more, more preferably 30% by weight or more, and even more preferably 60% by weight or more can be used. In addition, in the present technique, for example, a plant-based raw material containing starch of 95% by weight or less, preferably 90% by weight or less, more preferably 85% by weight or less, and even more preferably 80% by weight or less, 70% by weight or less, 60% by weight or less, 50% by weight or less, 40% by weight or less, 30% by weight or less, or 20% by weight or less can be used.

To summarize the above, it is preferable to use cereals, pulses excluding soybeans, and/or grains with a starch content of 10% by weight or more in the edible portion, as plant-based raw materials in which the plant-based viscous food and drink-producing enzyme preparation and the viscosity enhancer for plant-based viscous food and drink according to the present technique can be used.

The properties of the plant-based raw materials in which the plant-based viscous food and drink-producing enzyme preparation and the viscosity enhancer for plant-based viscous food and drink according to the present technique can be used are not particularly limited, as long as they do not impair the action and effect of the present technique, but preferably include liquid (including suspension) and slurry forms.

(4) Type of plant-based viscous food and drink

The type of the plant-based viscous food and drink that can be produced using the plant-based viscous food and drink-producing enzyme preparation according to the present technique, and the type of the plant-based viscous food and drink whose viscosity can be enhanced using the viscosity enhancer for plant-based viscous food and drink according to the present technique, are not particularly limited as long as they do not impair the action and effect of the present technique. The plant-based viscous food and drink-producing enzyme preparation according to the present technique, and the viscosity enhancer for plant-based viscous food and drink according to the present technique, can be preferably used in particular for plant-based fermented foods and drinks.

In the present technique, the term "plant-based fermented food and drink" refers to foods and drinks obtained by fermenting plant-based food and drink materials. Specific examples include plant-based fermented milk (plant-based yogurt), plant-based lactic acid bacteria drinks, plant-based yogurt paste, plant-based cheese-like foods, alcoholic drinks (sake, beer, wine, shochu, etc.), various fermented seasonings (soy sauce, miso, vinegar, etc.), rice bran pickles, kimchi, natto, kuzumochi, etc. In addition, in the present technique, the term "plant-based fermented food and drink" also includes secondary processed foods and drinks using these plant-based fermented foods and drinks. Among these, the present technique can be suitably used for plant-based lactic acid fermented food and drink, and can be suitably used for a plant-based yogurt in particular.

(5) Other

For the plant-based viscous food and drink-producing enzyme preparation and the viscosity enhancer for plant-based viscous food and drink according to the present technique, other enzymes and other ingredients can be used in combination, as long as the action and effect of the present technique are not impaired. Examples of other ingredients include excipients, pH adjusters, colorants, flavoring agents, disintegrants, lubricants, stabilizers, and the like that are normally used in formulations. Furthermore, ingredients with known or future functions can also be used in combination as appropriate depending on the purpose.

2. Method for producing plant-based viscous food and drink, and method for enhancing viscosity of plant-based viscous food and drink

The method for producing a plant-based viscous food and drink and the method for enhancing the viscosity of a plant-based viscous food and drink according to the present technique are methods that involve a step of allowing $\alpha$-amylase and cellulase to act on a plant-based raw material (hereinafter also referred to as an "enzyme action step"). In addition, a fermentation step, a recovery step, etc. can also be performed as necessary. Each step will be described in detail below.

(1) Enzyme action step

The enzyme action step is a step of allowing $\alpha$-amylase and cellulase to act on the plant-based raw material. In the enzyme action step, a step of allowing $\alpha$-amylase to act on the plant-based raw material (hereinafter also referred to as the "$\alpha$-amylase action step") and a step of allowing cellulase to act on the plant-based raw material (hereinafter also referred to as the "cellulase action step") can be carried out simultaneously or in any order.

(1-1) α-Amylase action step

Various conditions in the α-amylase action step can be freely set as long as they do not impair the effects of the present technique. For example, the pH, temperature, action time, etc. can be set according to the physicochemical properties of the α-amylase used, such as the optimum pH, stable pH range, optimum temperature, and temperature stability. The pH can be set, for example, to pH 4.0 to 10.0, pH 4.5 to 9.0, preferably pH 5.0 to 8.0, and more preferably pH 5.5 to 7.5. The temperature can be set, for example, to 10°C to 80°C, preferably 25°C to 75°C, 35°C to 70°C, and more preferably 50°C to 70°C. The action time can be set, for example, to 5 minutes to 48 hours, preferably 30 minutes to 24 hours, 1 to 12 hours, 2 to 8 hours, and more preferably 3 to 6 hours. The optimal reaction conditions can be determined through preliminary experiments.

(1-2) Cellulase action step

Various conditions in the cellulase action step can be freely set as long as they do not impair the effects of the present technique. For example, the pH, temperature, action time, etc. can be set according to the physicochemical properties of the cellulase used, such as the optimum pH, stable pH range, optimum temperature, and temperature stability. The pH can be set, for example, to pH 3.0 to 8.5, pH 3.5 to 8.0, preferably pH 4.0 to 7.0, and more preferably pH 4.5 to 6.5. The temperature can be set, for example, to 20°C to 75°C, preferably 25°C to 70°C, 25°C to 70°C, and more preferably 35°C to 65°C. The action time can be set, for example, to 5 minutes to 48 hours, preferably 30 minutes to 24 hours, 1 to 12 hours, 2 to 8 hours, and more preferably 3 to 6 hours. The optimal reaction conditions can be determined through preliminary experiments.

(2) Fermentation step

When a plant-based viscous food and drink produced using the method for producing a plant-based viscous food and drink of the present technique, or a plant-based viscous food and drink whose viscosity is to be enhanced using the method for enhancing the viscosity of a plant-based viscous food and drink of the present technique, is a plant-based fermented food and drink, a fermentation step can be performed.

The fermentation step is a step of fermenting a part or the whole of the plant-based raw material. The fermentation step can be carried out after the enzyme action step or can be carried out simultaneously with the enzyme action step.

In the fermentation step, fermentation can be performed using one or more types of microorganisms selected from anaerobic microorganisms, aerobic microorganisms, and facultative anaerobic microorganisms. As the microorganisms that can be used in the present technique, one or more types of microorganisms that can be used in the production of general fermented foods and drinks can be freely selected and used depending on the type of the plant-based fermented food and drink to be produced, as long as the effects of the present technique are not impaired. Examples of the microorganisms include lactic acid bacteria, yeast, filamentous fungi, Bacillus subtilis, and Acetobacteraceae.

In the fermentation step of the present technique, lactic acid fermentation can be performed. As the lactic acid bacteria used in lactic acid fermentation, one or more types of lactic acid bacteria that can be used in the production of general lactic acid fermented foods and drinks can be freely selected and used depending on the type and purpose of the lactic acid fermented food and drink to be produced, as long as the effect of the present technique is not impaired. Examples of lactic acid bacteria include lactic acid spherical bacteria belonging to Lactococcus, Streptococcus, Pediococcus, and Leuconostoc; lactic acid rod-shaped bacteria belonging to Lactobacillus; and Bifidobacterium, and preferably Lactobacillus gasseri, Lactobacillus rhamnosus, Bifidobacterium infantis, and Bifidobacterium adolescentis.

Various conditions for carrying out the fermentation step can be freely set as long as they do not impair the effects of the present technique. For example, fermentation conditions, heating conditions, etc. can be set according to the type of microorganism used. The fermentation temperature can be set, for example, to 20°C to 50°C, preferably 25°C to 45°C, more preferably 30°C to 40°C. The fermentation time can be set, for example, to 1 to 30 hours, preferably 2 to 20 hours, more preferably 4 to 10 hours. Examples of heating conditions include a high-temperature short-time sterilization (HTST) method, an ultra-high temperature cooking (UHT) method, a retort method, etc., and any condition may be used as long as the temperature of the food and drink material is 90°C or higher, preferably about 95°C. Examples thereof include a method of treating the food and drink material at 90 to 100°C for 1 to 5 minutes, or a method of treating the food and drink material at 90 to 95°C for 1 to 3 minutes.

(3) Recovery step

The recovery step is a step of recovering the plant-based viscous food and drink produced through an enzyme action step and, if necessary, a fermentation step. As for the specific recovery method, one or more of the recovery methods generally used in the production of a plant-based viscous food and drink can be freely combined and used depending on the type of the plant-based viscous food and drink to be produced.

One embodiment of the method for producing a plant-based viscous food and drink and the method for enhancing the viscosity of a plant-based viscous food and drink according to the present technique includes the following steps (1) and (2). An enzyme deactivation step may be added after step (2). The enzymes used in step (2) may be treated simultaneously or separately. When the enzymes are treated separately, the order of treatments is not particularly limited. When the enzymes are treated separately, an enzyme deactivation step may be added between each enzyme treatment as necessary.

(1) A step of preparing a plant-based raw material
(2) A step of allowing α-amylase and cellulase to act on the prepared plant-based raw material

One embodiment of the method for producing a plant-based viscous food and drink and the method for enhancing the viscosity of a plant-based viscous food and drink according to the present technique includes the following steps (1) to (3). An enzyme deactivation step may be added after step (2). The enzymes used in step (2) may be treated simultaneously or separately. When the enzymes are treated separately, the order of treatments is not particularly limited. When the enzymes are treated separately, an enzyme deactivation step may be added between each enzyme treatment as necessary.

(1) A step of preparing a plant-based raw material
(2) A step of allowing α-amylase and cellulase to act on the prepared plant-based raw material
(3) A step of recovering the plant-based viscous food and drink

One embodiment of the method for producing a plant-based viscous food and drink and the method for enhancing the viscosity of a plant-based viscous food and drink according to the present technique includes the following steps (1) to (4). Steps (2) and (3) may be performed simultaneously. Furthermore, an enzyme deactivation step may be added after steps (2) and/or (3). The enzymes used in step (2) may be treated simultaneously or separately. When the enzymes are treated separately, the order of treatments is not particularly limited. Furthermore, when the enzymes are treated separately, an enzyme deactivation step may be added between each enzyme treatment as necessary.

(1) A step of preparing a plant-based raw material
(2) A step of allowing α-amylase and cellulase to act on the prepared plant-based raw material
(3) A step of fermenting a part or the whole of the plant-based raw material
(4) A step of recovering the plant-based viscous food and drink

EXAMPLES

The present invention will be described in more detail below with reference to examples. Note that the examples described below are representative examples of the present invention, and the scope of the present invention should not be construed as being narrowed by these examples.

1. Raw material

The enzymes and plant-based raw materials used in the examples are shown in Table 1 below.

[Table 1]

| Name of substance | Product name | Distributors |
|---|---|---|
| Oat flour | Anthony's Organic Whole Grain Oat Flour Carbohydrate 22 g/32 g, dietary fiber 3 g/32 g | Anthony's |
| Tapioca starch | Tapioca starch | EAR THBORN Elements |
| Pea-derived protein | Pea protein 870 MV | PURIS |
| Yogurt starter | Vegan Starter Cultures for Homemade BIO Yogurt (microorganisms contained: Lactobacilus Gasseri, Lactobacilus Rhamnosus, Bifidobacterium infantis, Bifidobacterium adolescentis) | Natural Probiotic Selection |

(continued)

| Name of substance | Product name | Distributors |
|---|---|---|
| α-amylase | Bacillus amyloliquefaciens-derived amylase | Amano Enzyme Inc. |
| Cellulase | Trichoderma viride-derived cellulase | Amano Enzyme Inc. |
| Hemicellulase | Aspergillus niger-derived hemicellulase | Amano Enzyme Inc. |
| Pectinase | Aspergillus pulverulentus-derived pactinase | Amano Enzyme Inc. |

2. Enzyme activity measurement method

[Method for measuring α-amylase activity]

10 mL of 1% potato starch dissolved in 1M acetic acid/sodium acetate buffer (pH 5.0) was accurately measured, and after heating at 37°C for 10 minutes, 1 mL of a sample solution containing α- amylase was accurately added and immediately shaken. After leaving this solution at 37°C for exactly 10 minutes, 1 mL of this solution was accurately measured, added to 10 mL of 0.1M hydrochloric acid test solution, and immediately shaken to stop the reaction. Next, 0.5 mL of this solution was accurately measured, 10 mL of 0.002M iodine test solution (Japanese Pharmacopoeia) was accurately added, and after shaking, the absorbance at 660 nm was measured using water as a control. Separately, 1 mL of water was accurately added instead of the sample solution, and the same operation was performed to measure the absorbance. Under these conditions, the amount of enzyme that reduces the color of potato starch by iodine by 10% in 1 minute was defined as 1 unit, and was calculated using the following formula.

$$\alpha\text{-Amylase activity (U/g, U/mL)} = \{(AB-AT)/AB\} \times 1/W$$

AT: absorbance of sample solution
AB: absorbance of blank solution
W: amount of sample in 1 mL of sample solution (g or mL)

[Method for measuring cellulase activity]

Cellulase activity can be measured by a method based on the cellulase activity test method in the 9th edition of Japan's Specifications and Standards for Food Additives.

4 mL of 0.625% carmellose (also known as sodium carboxymethylcellulose) dissolved in 1M acetic acid/sodium acetate buffer (pH 4.5) was accurately measured and placed in a 50 mL Nessler tube, and left at 37°C for 10 minutes or more, after which 1 mL of a sample solution containing cellulase was accurately measured and added, and immediately shaken. This solution was left at 37°C for exactly 30 minutes, 2 mL of alkaline copper test solution was accurately added and shaken, 3 mL of 0.5M sodium hydroxide test solution was accurately added, the precipitate was dissolved by shaking, and after leaving for 20 minutes, 13 mL of 1M acetic acid/sodium acetate buffer solution (pH 4.5) was accurately added. 1 mL of this solution was accurately measured, 9 mL of 1M acetic acid/sodium acetate buffer solution (pH 4.5) was accurately added, and the solution was shaken well. The absorbance of this solution at a wavelength of 750 nm was measured using water as a control. Separately, 1 mL of a sample solution was accurately measured and placed in a 50 mL Nessler tube, 2 mL of alkaline copper test solution was accurately added and shaken, then 4 mL of a substrate solution was accurately measured and added, shaken, and the absorbance was measured in the same manner. Under these conditions, the amount of enzyme that brings about an increase in reducing power equivalent to 1 μmole of glucose per minute was defined as 1 unit, and was calculated using the following formula.

$$\text{Cellulase activity (U/g, U/mL)} = (A1 - A2/a) \times (1/30) \times (1/0.180) \times n$$

A1: absorbance of sample solution
A2: absorbance of blank solution
a: slope of glucose calibration curve

1/30: conversion factor to values per minute
1/0.180: 1 μmole of glucose = 0.180 mg
n: dilution factor per 1 g or 1 mL of sample

3. Experimental example

<Experimental Example 1>

In Experimental Example 1, the influence on the viscosity and water retention of a plant-based food due to differences in the enzymes used was investigated. In Experimental Example 1, oat flour (powder) was used as an example of a plant-based raw material, and a plant-based yogurt was produced as an example of a plant-based viscous food and drink.

(1) Production of plant-based yogurt

7.5 g of oat flour was added to 42.5 g of purified water, and the mixture was mixed at 60°C for 30 minutes at a speed of 400 rpm using a heater-equipped stirrer. Next, the enzymes shown in Table 2 were added and stirred for another 1 hour. After the reaction was completed, 1.5 g of tapioca starch, 1.5 g of pea-derived protein, and 0.2 g of tricalcium phosphate were added, and the mixture was mixed at 60°C for 10 minutes at a speed of 400 rpm using a heater-equipped stirrer. Then, the mixture was treated in a water bath at 95°C for 20 minutes. The mixture was then cooled at room temperature, and kept at 40°C for 30 minutes at a speed of 400 rpm using a heater-equipped stirrer, and 10 mg of yogurt starter was suspended in 100 μL of sterilized water and added. Then, the mixture was left to ferment in a constant temperature bath at 40°C, and the fermentation was stopped after 24 hours by moving it to a refrigerator, and a plant-based yogurt was produced.

(2) Measurement and evaluation

The viscosity and water retention of the produced plant-based yogurt were measured and evaluated by the following methods.

[Viscosity]

35 ml of yogurt was transferred to a 50 ml tube, and the viscosity was measured using a Brookfield Digital Viscometer (Model DV-E) according to the method described in the instruction manual for the Brookfield viscometer. The spindle used was model S61, the rotation speed was set to 30 rpm, and the sample temperature was kept constant at around 20°C. A stable value was confirmed 2 minutes after the spindle started to rotate. The average value of the two measurements was used.

[Water retention]

10 g of yogurt was placed in a 15 mL tube and centrifuged at 600 × g for 20 minutes in a centrifuge, after which the supernatant was collected and weighed. Water retention was calculated according to the following formula. Improved water retention means an increase in the water content (increase in water retention capacity) in the resulting plant-based yogurt.

$$\text{Water retention (\%)} = (1 - (\text{weight of supernatant/weight of sample})) \times 100$$

(3) Result

The results are shown in Table 2.

[Table 2]

| | | | Comparative Example | | | Example |
|---|---|---|---|---|---|---|
| | | | 1 | 2 | 3 | 1 |
| Enzyme used | α-amylase | U/g-oat flour | 98 | 98 | 98 | 98 |
| | Cellulase | U/g-oat flour | - | - | - | 21 |
| | Hemicellulase | U/g-oat flour | - | 67500 | - | - |
| | Pectinase | U/g-oat flour | - | - | 90 | - |
| Evaluation | Viscosity | mPas/30 rpm | 44 | 57 | 57 | 200 or more |
| | | Ratio to no cellulase | - | 1.3 | 1.3 | 4.5 or more |
| | Water retention | % | 47 | 46 | 48 | 56 |
| | | Ratio to no cellulase | - | 1.0 | 1.0 | 1.2 |

(4) Discussion

As shown in Table 2, in comparison with Comparative Example 1 in which only α-amylase was used, Comparative Example 2 in which hemicellulase was used in combination and Comparative Example 3 in which pectinase was used in combination had slightly enhanced viscosity but equivalent water retention. On the other hand, in Example 1 in which α-amylase and cellulase were used in combination, an accurate measurement value could not be obtained because the viscosity exceeded the upper limit, but compared with Comparative Examples 1 to 3, the viscosity was significantly enhanced and water retention was also improved.

<Experimental Example 2>

In Experimental Example 2, the effect of the difference in the amount of cellulase added on the viscosity and water retention of a plant-based viscous food and drink was examined. In Experimental Example 2, oat flour was used as an example of a plant-based raw material, and a plant-based yogurt was produced as an example of a plant-based viscous food and drink.

(1) Production of plant-based yogurt

A plant-based yogurt was produced in the same manner as in Experimental Example 1 using the enzymes shown in Table 3 below.

(2) Measurement and evaluation

The viscosity and water retention of the produced plant-based yogurt were measured and evaluated by the following methods.

[Viscosity]

Since the viscosity exceeded the upper limit in Example 1 of Experimental Example 1, the rotation speed was set to 5 rpm in Experimental Example 2. The viscosity was measured in the same manner as in Experimental Example 1, except for the rotation speed.

[Water retention]

The water retention was evaluated in the same manner as in Experimental Example 1.

(3) Result

The results are shown in Table 3.

[Table 3]

| | | | Comparative Example | Example | | | | |
|---|---|---|---|---|---|---|---|---|
| | | | 4 | 2 | 3 | 4 | 5 | 6 |
| Enzyme used | α-amylase | U/g-oat flour | 98 | 98 | 98 | 98 | 98 | 98 |
| | Cellulase | U/g-oat flour | - | 105 | 42 | 10.5 | 2.1 | 1.1 |
| Evaluation | Viscosity | Ratio to no cellulase | 1.0 | 3.2 | 5.8 | 3.0 | 1.9 | 1.4 |
| | Water retention | Ratio to no cellulase | 1.0 | 1.4 | 1.2 | 1.1 | 1.0 | - |

(4) Discussion

As shown in Table 3, it was found that the viscosity was enhanced regardless of the amount of cellulase added. In addition, since the viscosity enhancing effect of Example 6 was higher than that of Comparative Examples 2 and 3 of Experimental Example 1, it was found that even an added amount of cellulase of 1.1 U/g-oat powder was sufficient to exhibit the viscosity enhancing effect.

It was found that the more the amount of cellulase added, the more the water retention improved. It was also shown that in order to ensure the water retention effect, it is preferable to use cellulase at a concentration of 10.5 U/g oat powder or more.

**Claims**

1. A plant-based viscous food and drink-producing enzyme preparation, comprising α-amylase and cellulase.

2. The plant-based viscous food and drink-producing enzyme preparation according to claim 1, wherein the plant-based viscous food and drink is a plant-based fermented food and drink.

3. The plant-based viscous food and drink-producing enzyme preparation according to claim 2, wherein the plant-based fermented food and drink is a plant-based lactic acid fermented food and drink.

4. The plant-based viscous food and drink-producing enzyme preparation according to claim 3, wherein the plant-based lactic acid fermented food and drink is a plant-based yogurt.

5. A viscosity enhancer for plant-based viscous food and drink, comprising α-amylase and cellulase.

6. A method for producing a plant-based viscous food and drink, comprising a step of allowing α-amylase and cellulase to act on a plant-based raw material.

7. A method for enhancing the viscosity of a plant-based viscous food and drink, comprising a step of allowing α-amylase and cellulase to act on a plant-based raw material.

## INTERNATIONAL SEARCH REPORT

| International application No. |
| --- |
| **PCT/JP2024/004985** |

### A. CLASSIFICATION OF SUBJECT MATTER

*A23L 5/00*(2016.01)i; *A23C 11/00*(2006.01)i; *C12N 9/28*(2006.01)i; *C12N 9/42*(2006.01)i; *A23L 7/104*(2016.01)n
FI:   A23L5/00 J; C12N9/42; C12N9/28; A23C11/00; A23L7/104

According to International Patent Classification (IPC) or to both national classification and IPC

### B. FIELDS SEARCHED

Minimum documentation searched (classification system followed by classification symbols)

A23L5/00; A23C11/00; C12N9/28; C12N9/42; A23L7/104

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | JP 2020-519245 A (THE QUAKER OATS COMPANY) 02 July 2020 (2020-07-02) claims 1, 30, paragraphs [0140], [0148] | 1-7 |
| X | WO 2021/256507 A1 (GODO SHUSEI KK) 23 December 2021 (2021-12-23) claims, paragraphs [0038], [0099]-[0101] | 1-7 |
| X | JP 2016-93151 A (LA VIE EN SANTE KK) 26 May 2016 (2016-05-26) claims, example 6 | 1-4, 6 |
| A | | 5, 7 |
| X | JP 2015-173623 A (TAIYO KAGAKU CO., LTD.) 05 October 2015 (2015-10-05) claims, example 1 | 1-4, 6 |
| A | | 5, 7 |

✓ Further documents are listed in the continuation of Box C.     ✓ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **16 April 2024** | **07 May 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

International application No.

**PCT/JP2024/004985**

### C. DOCUMENTS CONSIDERED TO BE RELEVANT

| Category* | Citation of document, with indication, where appropriate, of the relevant passages | Relevant to claim No. |
|---|---|---|
| X | CN 111631268 A (HENAN DINGDANGNIU FOOD CO., LTD.) 08 September 2020 (2020-09-08)<br>        claims, example 1 | 1-4, 6 |
| A | | 5, 7 |
| P, X | JP 2023-146935 A (OTSUKA FOODS CO., LTD.) 12 October 2023 (2023-10-12)<br>        claims, test examples | 1, 6 |
| P, A | | 2-5, 7 |
| P, X | JP 2023-168704 A (HBI KK) 29 November 2023 (2023-11-29)<br>        claims | 1, 6 |
| P, A | | 2-5, 7 |
| A | CN 112438324 A (YOUYUANCHUANG (XIAMEN) BIO-TECH CO., LTD.) 05 March 2021 (2021-03-05)<br>        entire text | 1-7 |

Form PCT/ISA/210 (second sheet) (July 2022)

EP 4 666 867 A1

## INTERNATIONAL SEARCH REPORT
### Information on patent family members

International application No.

**PCT/JP2024/004985**

| Patent document cited in search report | | | Publication date (day/month/year) | Patent family member(s) | | | Publication date (day/month/year) |
|---|---|---|---|---|---|---|---|
| JP | 2020-519245 | A | 02 July 2020 | US | 2018/0327792 | A1 | |
| | | | | claims 1, 30, paragraphs [0146], [0154] | | | |
| | | | | WO | 2018/226358 | A1 | |
| | | | | EP | 3621460 | A1 | |
| | | | | KR | 10-2020-0007009 | A | |
| | | | | CN | 110621167 | A | |
| WO | 2021/256507 | A1 | 23 December 2021 | US | 2023/0292778 | A1 | |
| | | | | claims, paragraphs [0118], [0220]-[0228] | | | |
| | | | | EP | 4166000 | A1 | |
| | | | | CN | 115835779 | A | |
| JP | 2016-93151 | A | 26 May 2016 | (Family: none) | | | |
| JP | 2015-173623 | A | 05 October 2015 | (Family: none) | | | |
| CN | 111631268 | A | 08 September 2020 | (Family: none) | | | |
| JP | 2023-146935 | A | 12 October 2023 | (Family: none) | | | |
| JP | 2023-168704 | A | 29 November 2023 | (Family: none) | | | |
| CN | 112438324 | A | 05 March 2021 | (Family: none) | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2022037920 A
- WO 2017171601 A
- WO 2022014542 A

### Non-patent literature cited in the description

- Manufacture and characterization of a yogurt-like beverage made with oat flakes fermented by selected lactic acid bacteria. *International Journal of Microbiology*, 2014, vol. 185, 17-26